# EUROPEAN PATENT SPECIFICATION

(11) **EP 1 848 445 B1**
(45) Date of publication and mention of the grant of the patent: **01.04.2015**
(21) Application number: 06805091.3
(22) Date of filing: 27.10.2006
(51) Int. Cl.: A61K 31/704, A61K 31/7024, A61P 9/10, A61P 9/00

(54) **PHARMACEUTICAL COMPOSITION AND METHOD FOR NEOANGIOGENESIS/REVASCULARIZATION USEFUL IN TREATING ISCHEMIC HEART DISEASES**
PHARMAZEUTISCHE ZUSAMMENSETZUNG UND VERFAHREN ZUR NEOANGIOGENESE/REVASKULARISIERUNG BEI DER BEHANDLUNG ISCHÄMISCHER HERZLEIDEN
COMPOSITION PHARMACEUTIQUE ET PROCEDE DE NEOANGIOGENESES/REVASCULARISATION UTILISE DANS LE TRAITEMENT DE MALADIES CARDIAQUES ISCHEMIQUES

(30) Priority: 27.10.2005 WO PCT/IB2005/003202; 27.10.2005 WO PCT/IB2005/003191; 13.04.2006 US 791462 P
(43) Date of publication of application: 31.10.2007
(73) Proprietor: Lead Billion Limited, Kowloon, Hong Kong (CN)
(72) Inventor: LI, Ming, Kowloon Hong Kong (CN); CHENG, Lei, The Chinese University Of Hong Kong, Shatin Hong Kong (CN); LIU, Hongwei, Taipo Hong Kong (CN)
(74) Representative: Savic, Bojan
(86) International application number: PCT/CN2006/002886
(87) International publication number: WO 2007/048353

(56) References cited:
- WO-A-03/043645
- WO-A-2004/052381
- CN-A- 1 558 769
- YOKOZAWA TAKAKO ET AL: "Potential of sanguiin H-6 against oxidative damage in renal mitochondria and apoptosis mediated by peroxynitrite in vivo" NEPHRON, vol. 92, no. 1, September 2002 (2002-09), pages 133-141, XP008123273 ISSN: 0028-2766
- DATABASE WPI Week 29, Derwent Publications Ltd., London, GB; AN 1987-201982, XP003012460 & JP 62 129 220 A (LEAD CHEM CO LTD) 11 June 1987
- MING D-S. ET AL.: 'Research Progress in Chemical Constituents and Biological Activities of Geum Species' ACTA PHARMACEUTICAL SINICA vol. 35, no. 7, 2000, pages 552 - 558, XP009069342
- LAI Y. ET AL.: 'Protective Effect of Aleppo Avens on Mouse Cerebral Ischemia' JOURNAL OF DALI UNIVERSITY vol. 4, no. 5, September 2005, XP008080311

## Description

### FIELD OF THE INVENTION

This invention relates to a pharmaceutical composition in relation to its use in a method of treating ischemic heart diseases. Particularly, it relates to a pharmaceutical composition and method for growing new blood vessels that supply oxygen and nutrients to infarcted heart tissues throughout the entire infarct zone and for preventing cardiomyocyte apoptosis in ischemic events.

### BACKGROUND OF THE INVENTION

Ischemic heart diseases including coronary heart disease and heart infarction are diseases due to insufficient coronary blood supply or interruption of the blood supply to a part of the heart, causing damages or death of heart muscle cells. It is the leading cause of death for both men and women over the world. For example, about 1.5 million Americans suffer a heart attack each year (that's about one heart attack every 20 seconds) and millions suffer from ischemic heart diseases.

During remodeling progress post infarction, neoangiogenesis/revascularization to the infarcted heart tissues is insufficient to keep pace with the tissue growth required for contractile compensation and is unable to support the greater demands of the hypertrophied but viable myocardium, especially the myocardium along the border zone of the infarct-the cardiomyocytes at risk. The relative lack of oxygen and nutrients to the hypertrophied myocytes might be an important etiological factor in the death of otherwise viable myocardium, resulting in progressive infarct extension and fibrous replacement. Therefore, the most direct way to rescue the cardiac myocytes at risk apparently is to establish a new blood supply at an early stage that would allow circulating stem cells, nutrients and growth factors, in addition to oxygenation, to be delivered to the infarct zone. Restoration of coronary blood flow by rapid angiogenesis should offer a direct and effective therapeutic modality to intractable ischemic heart diseases.

Although therapeutic angiogenesis has been studied intensively as an alternative treatment for ischemic vascular diseases using growth factors such as VEGF, aFGF, bFGF or PDGF, these factors take weeks to act¹⁻⁶, while myocardial necrosis due to coronary occlusion occurs very rapidly within a matter of hours ^{5,7,8}. The consequence is that fibrous tissue grows rapidly despite the ischemic condition, which replaces the infarcted heart tissues and leaves little room for any newly regenerated myocyte replacement. Up to now, there is no drug and therapeutic method available that can promote early reconstitution of the damaged coronary vasculature with newly formed vessels.

Therefore, to realize the therapeutic value of angiogenesis in combating ischemic heart diseases, there is a need for chemical compounds possessing biological properties that can sufficiently promote early growth of new blood vessels in the infarct zone to quickly restore the coronary blood circulation once an ischemic event occurs.

PCT application WO 03/043645 relates to an organic extract of *Geum Japonicum Thunb,* comprising a mixture of several hydrolyzable tannins (such as Gemin A, B, C, D, E and F), among others, for treating ischemic and infracted heart muscles by stimulation of both angiogenesis and myocardial regeneration at early stage of the healing process of damaged tissue.

PCT application WO 2004/052381 relates to an organic extract of *Geum Japonicum Thunb* variant, comprising a mixture of several hydrolyzable tannins (such as Gemin A, B, C, D, E and F), among others, for treating tissue damage or ischemia (e.g. following muscle or skin injury and bone fracture).

### SUMMARY OF THE INVENTION

As one object of the present invention, there is provided a pharmaceutical composition for treating ischemic heart diseases which comprises an isolated compound of formula (I). The compound of formula (I) has shown potent beneficial therapeutic effects in treating ischemic heart diseases by promoting angiogenesis and protecting against endothelial apoptosis, resulting in revascularization in infarcted myocardia and prevention of further ischemic death of the cardiomyocytes. Said compound is referred to as "Ga" hereinafter. The compound is known in the art but it has never known as possessing the above biological activities and therapeutic effects. In fact, the tannins, to which Ga belongs, are conventionally reviewed as non-active ingredients and in the process of identifying the active ingredients in herbal medicines researchers routinely discard the tannins as debris. Ga may be isolated from natural resources, particularly from plants or they may, with existing or future developed synthetic techniques, be obtained through total or semi-chemical syntheses.

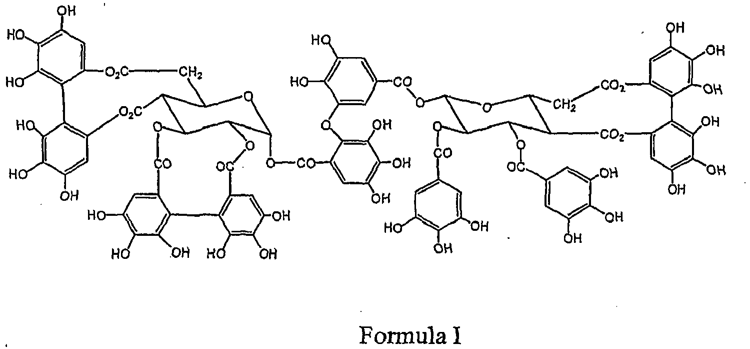

The backbone compound of formula I (also referred to as Ga in this application) can have substituents at various positions and retain similar biological activities as the backbone compound Ga. A substituent is an atom or group of atoms substituted in place of the hydrogen atom. The substitution can be achieved by methods known in the field of organic chemistry.

A person with ordinary skill in the art would contemplate, that the above backbone compound or its substituted variant may be made in various possible racemic, enantiomeric or diastereoisomeric isomer forms, may form salts with mineral and organic acids, and may also form derivatives such as N-oxides, prodrugs, bioisosteres. "Prodrug" means an inactive form of the compound due to the attachment of one or more specialized protective groups used in a transient manner to alter or to eliminate undesirable properties in the parent molecule, which is metabolized or converted into the active compound inside the body (in vivo) once administered. "Bioisostere" means a compound resulting from the exchange of an atom or of a group of atoms with another, broadly similar, atom or group of atoms. The objective of a bioisosteric replacement is to create a new compound with similar biological properties to the parent compound. The bioisosteric replacement may be physicochemically or topologically based. Making suitable prodrugs, bioisosteres, N-oxides, pharmaceutically acceptable salts or various isomers from a known compound (such as those disclosed in this specification) are within the ordinary skill of the art. Therefore, all suitable isomer forms, salts and derivatives of the above disclosed compounds are disclosed herein.

As disclosed herein, the term "functional derivative" means a prodrug, bioisostere, N-oxide, pharmaceutically acceptable salt or various isomer from the above-disclosed specific compound, which may be advantageous in one or more aspects compared with the parent compound. Making functional derivatives may be laborious, but some of the technologies involved are well known in the art. Various high-throughput chemical synthetic methods are available. For example, combinatorial chemistry has resulted in the rapid expansion of compound libraries, which when coupled with various highly efficient bio-screening technologies can lead to efficient discovering and isolating useful functional derivatives.

The pharmaceutical composition may be formulated by conventional means known to people skilled in the pharmaceutical industry into a suitable dosage form, such as tablet, capsules, injection, solution, suspension, powder, syrup, etc, and be administered to a mammalian subject suffering coronary heart disease or myocardial infarction (MI) in a suitable manner.

In another aspect, the present invention provides (1) a method of promoting revascularization in dead or damaged heart tissues caused by an ischemic heart disease, such as, for example, atherosclerosis of coronary arteries in a mammalian subject. The method comprises a step of administering an effective amount of the compound of formula (I) to the mammalian subject.

In still another aspect, present invention provides (1) a method for treating, ameliorating or curing a pathological condition in a mammal, where the pathological condition, as judged by people skilled in medicine, can be treated or alleviated by up-regulating the expressions of angiogenic factors (VEGF and FGF) that promotes early revascularization in infarcted myocardium, and/or by inducing anti-apoptotic protein expression that inhibits apoptotic death of cardiomyocytes in the infarcted hearts and prevents the progressive extending of further ischemic injury and limiting infarct size. The method comprises a step of administering an effective (1)<an isolated compound of formula (I) for use in> amount of the compound of formula (I) to the mammal.

The various features of novelty which characterize the invention are pointed out with particularity in the claims annexed to and forming a part of this disclosure. For a better understanding of the invention, its operating advantages, and specific objects attained by its use, reference should be made to the drawings and the following description in which there are illustrated and described preferred embodiments of the invention.

### BRIEF DESCRIPTION OF DRAWINGS

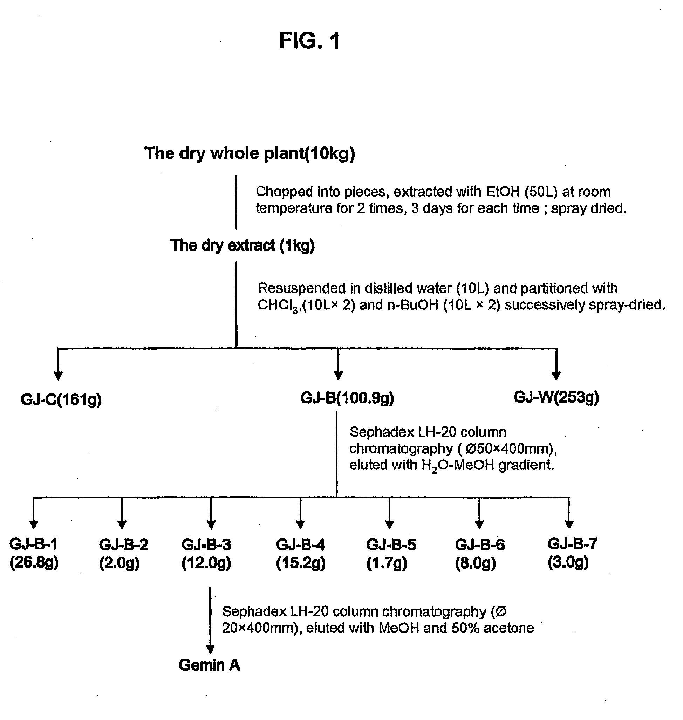

FIG. 1 outlines the process of isolating Ga from the plant of *Geum Japonicum* as an example of making the compound of the present invention.

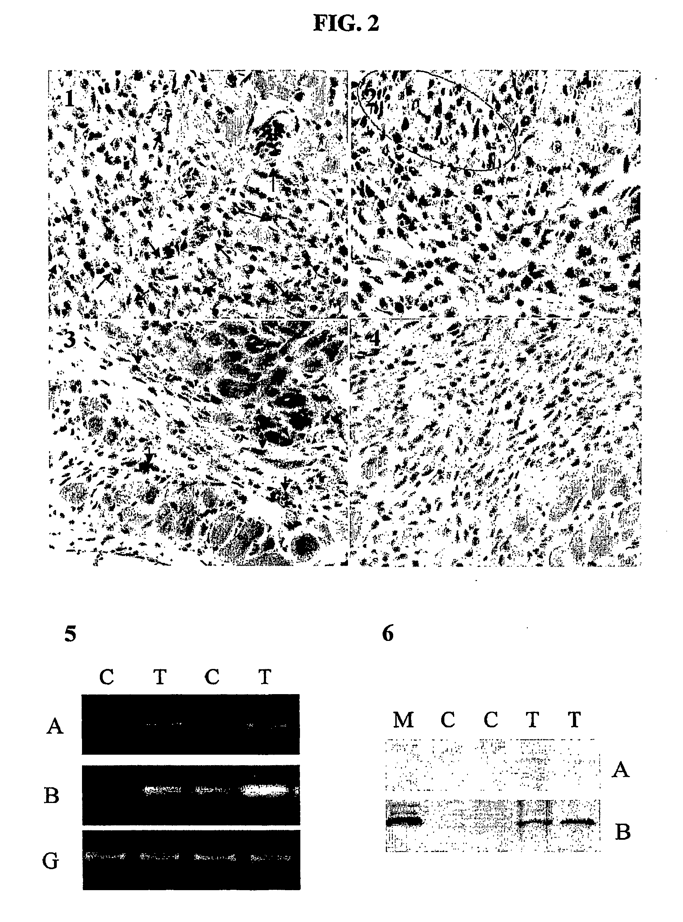

FIG. 2. shows the effect of early neovascularization of the infarcted myocardium following Ga treatment. **1:** two days after left anterior descending coronary artery (LAD) ligation and Ga injection; **2:** two day control heart; **3:** seven days after LAD ligation and Ga injection; **4:** seven days control heart; **5:** RT-PCR analysis and **6:** Western blot analysis, showing significantly up-regulated gene expressions of VEGFb and VEGFc in the Ga treated heart tissues (A standing for VEGFb, B for VEGFc, G for GAPDH, C for control group, T for Ga treated group, M for molecular marker).

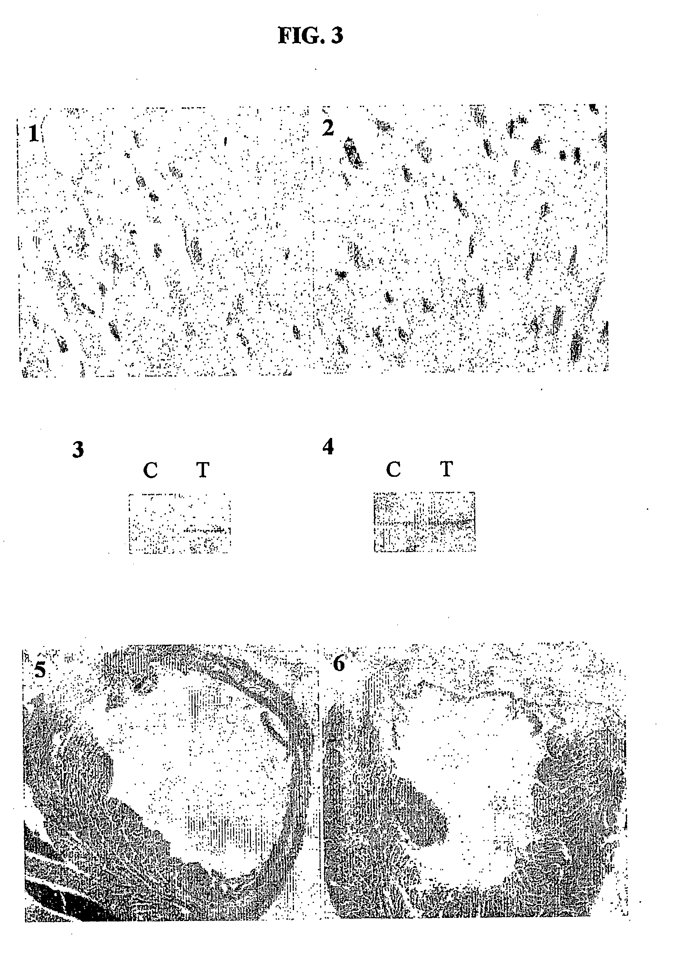

FIG. 3 shows the Ga-induced effect on survival potential and infarct size. **1:** seven days after LAD ligation (control); **2:** seven days after LAD ligation (Ga treated); **3:** Western blot analysis showing increased expressions of phospho-Akt1 with Ga treatment; **4:** Western blot analysis showing increased expressions of Bcl2 with Ga treatment (C and T standing for control group and Ga treated group, respectively); **5:** trichrome staining of the rat myocardium at 2-week post infarct (control); and **6:** trichrome staining of the rat myocardium at 2-week post infarct (Ga treated), showing significantly reduced infarct size and increased mass of viable myocardium within the anterior wall.

### DETAILED DESCRIPTION OF PARTICULAR EMBODIMENTS

### I. Experimental Procedures

All protocols used in the present invention conformed to the Guide for the Care and Use of Laboratory Animals published by the U.S. National Institutes of Health, and were approved by the Animal Experimental Ethical Committee of The Chinese University of Hong Kong.

*Isolation of Ga from Geum Japohicum:* For the experiments disclosed in the following, Ga was obtained from the plant of *Geum Japonicum*. Referring to **FIG.1****,** the plant was collected from Guizhou Province of China in August was dried (10kg) and percolated with 70% ethanol (100L) at room temperature for 3 days twice. The extract was combined and spray-dried to yield a solid residue (1kg). The solid residue was suspended in 10 liter H₂O and successively partitioned with chloroform (10 L) twice, then n-butanol (10 L) twice to produce the corresponding fractions. The n-butanol (GJ-B) soluble fraction was filtered and spray dried to yield a powder fraction. It was shown that n-BuOH soluble fraction could significantly enhance the proliferation of HCAECs-human coronary artery endothelial cells (Clonetics, Inc.) and stimulate rapid neovascularization in infarct zone of MI animal model. The n-BuOH soluble fraction was applied on a column of Sephadex LH-20 equilibrated with 10% methanol and eluted with increasing concentration of methanol in water, resolving 7 fractions. Fraction 3, eluted with approximately 50% methanol, showed the potent activity in stimulating significant angiogenesis in infarcted myocardium. This fraction 3 containing tannins was used to test its healing effects on a MI animal model. The structures of the active compounds contained in this active fraction were determined by NMR analysis. Of course, as Ga is a known natural occurring compound, it may be obtained from other plants and produces satisfactory results.

*Animals, surgical procedures:* Male Sprague-Dawley (SD) rats, weighing 250-300 g were used. Following proper anesthesia, a left thoracotomy was performed on the animals, the pericardium was opened and the left anterior descending (LAD) coronary artery was ligated. Ga dissolved in PBS (0.1ml, containing 0.3mg Ga) was injected into the distal myocardium (the presumed ischemic region) of the ligated artery immediately after the ligation in the test group (having 60 rats, i.e., n=60). An equivalent volume of PBS was injected to the corresponding location of the rats in the control group (n=60). Fifteen rats of each group were euthanatized on day 2, 7, 14 and 30 post-infarct for morphological and functional assessment. For the sham group (n=6), left thoracotomy was performed and the pericardium was opened but with no LAD ligation. For the normal control group (n=6), the rats were not subject o any surgical procedures and treatments.

*Measurement of neovascularization in the infarct zone:* Left ventricles from the rats sacrificed on day 2 and 7 post-infarction were removed and sliced from apex to base in 3 transverse slices. The slices were fixed in formalin and embedded in paraffin. Vascular density was determined on the histology section samples by counting the number of vessels within the infarct zone using a light microscope under a high power field (HPF) (× 400). Eight random and non-overlapping HPFs within the infarct filed were used for counting all the vessels in each section. The number of vessels in each HPF was averaged and expressed as the number of vessels per HPF. Vascular counts were performed by two investigators in a blind fashion.

*Measurement of myocyte apoptosis by TUNEL assay of paraffin tissue sections:* The TUNEL assay method was used for *in situ* detection of apoptosis at the single-cell level⁹. Rat myocardial infarction tissue sections were obtained from both the test group and the control group on day 7 post-infarction. After general deparaffinization and rehydration, tissues were digested with Proteinase K (Dako) for 15 minutes and incubated with TdT (Roche) and Biotin-16-dUTP (Roche) for 60 minutes at 37°C. After incubation with SP-HRP (Roche) for 20 minutes, the TUNEL staining was visualized with DAB (Dako), which stained the nuclei (with DNA fragmentation stained brown). Tissue sections were examined microscopically at a high power field (× 400) and at least 100 cells were counted in a minimum of 10 HPF. The number of the apoptotic myocytes per HPF was referred to as the apoptotic index.

*Estimation of the myocardial infarction:* The hearts of the rats, sacrificed on day 14 post infarction, were removed and sectioned from apex to base in three to four transverse slices and embedded in paraffin. Thin sections (5 µm thick) were cut from each slide and stained with H&E staining and Masson's trichrome (Sigma, USA), which labels collagen blue and myocardium red. These sections from all slices were projected onto a screen for computer-assisted planimetry (ImageJ 1.34S, Wayne Rasband, National Institutes of Health, USA). The endocardial and epicardial circumferences as well as the length of the scar were measured for each slice. The infarcted portion of the left ventricle was calculated from these measurements and the ratio of scar length to ventricular circumference of the endocardium and epicardium of the slices was expressed as a percentage to define the infarct size ^{9.10,11}.

*Echocardiography Assessment of Myocardial Function:* In all, 118 SD rats received baseline echocardiography before any experimental procedures. Echocardiography was recorded under controlled anesthesia using a S10-MHz phased-array transducer and GE VingMed Vivid 7 system. M-mode tracing and 2-dimensional (2D) echocardiography images were recorded from the parasternal long- and short-axis views. Short axis view was at the papillary muscles level. Left ventricular end-diastolic (LVDA) and end-systolic (LVSA) areas were planimetered from the parasternal long axis and LV end-diastolic and end-systolic volumes (LVEDV and LVESV) were calculated by the M-mode method. LV ejection fraction (LVEF) and fractional shortening (FS) were derived from LV cross-sectional area in 2D short axis view: *EF* = [(LVEDV - LVESV)/LVEDV] x 100% and *FS =* [(LVDA - LVSA)/LVDA] x 100% ¹². Standard formulae were used for echocardiographic calculations.

*RT-PCR analysis of survival associated gene expressions:* A small slice from the above prepared infarcted myocardial tissue were put into liquid nitrogen immediately after incision and stored at -80°C. According to manufacturer's instructions, total RNA was isolated using Qiagen RNeasy Mini Kit (Catalog Number 74104, Qiagen, Germany), dissolved in 20-30µl RNase free water and stored at -80°C. The integrity of the ribosomal RNA and DNA contamination was checked routinely using formaldehyde denaturing RNA gel electrophoresis (1.2%) before proceeding with the further analysis. Protein contamination and concentration of the total RNA was assessed by determining the ratio OD260:OD280 spectrophotometrically (Eppendorf BioPhotometer, Hamburg, Germany).

*Western Blot Analysis:* About 50mg of the above prepared infarcted myocardial tissue were grinded to powder in liquid nitrogen. 1 mL lysis buffer (50 mM Tris, pH 7.5, 150 mM NaCl, 1 mM EDTA, 1 mM EGTA, 1% Nonidet P-40, 10% glycerol, 200 mM NaF, 20 mM sodium pyrophosphate, 10 mg/ml leupeptin, 10 mg/ml aprotinin, 200 mM phenylmethylsulfonyl fluoride, and 1 mM sodium orthovanadate) was added to the powder and put on ice for 30 min. Protein yield was quantified by Bio-Rad DC protein assay kit (Bio-Rad). Equal amounts (10 g) of total protein were size-fractionated by SDS-PAGE and transferred to PVDF membranes (Amersham, USA). The blots were blocked with phosphate-buffered saline plus 0.1% (vol/vol) Tween 20 (PBST) containing 5% (wt/vol) milk powder (PBSTM) for 30 min at room temperature and probed for 60 min with specific primary antibodies against rat phospho-Akt1 (mouse, Santa Cruz) or rat Bcl-2 (mouse, Sigma-Aldrich), diluted 1:1000 in PBSTM. After washing extensively in PBST, the blots were probed by horseradish peroxidase-coupled antimouse IgG (Amersham Biosciences) (1/1000 dilution in PBSTM, 60 min), extensively washed with PBST, and developed by chemiluminescence.

*Biostatistics:* All morphometric data were collected blindly. Results are presented as mean ±SD computed from the average measurements obtained from each heart. Statistical significance for comparison between two measurements was determined using the unpaired two-tailed Student's t test. Values of P<0.05 were considered to be significant.

### II. Ga-induced revascularization in Infarcted myocardium

Referring to **FIG. 2****,** histology studies revealed that many vessels were newly formed throughout the entire infarct zone, including the central areas and the border zones on day 2 post infarction (**FIG. 2****:****1**), where the newly formed vessels are pointed to by red arrowheads). Some of the newly formed vessels were filled with blood cells and others were still at the early stage of the vessel regeneration development and displayed as a lumen like structure without filling of blood cells. The capillary density in the infarct zone of the Ga treated myocardium was on average 18 (18±3.9) filling with blood cells and 8 (8 ±2.8) lumen-like structures per HPF, calculated from 8 randomly selected view fields on each of the 15 slides from 15 Ga treated hearts on day 2 (**FIG. 2****:** **1**). By contrast, fewer blood vessels (5±2.1 per HPF) with an inflammatory cell infiltration were observed in the infarct zone in the control myocardium on day 2 post MI (**FIG. 2: 2**). In Ga treated hearts, on day 7 post MI, the newly formed blood vessels filled with blood cells remained (11±3.6) throughout the infarct zone but the lumen-like structures were not observed (**FIG. 2****:** **3**). By contrast, the control samples showed mainly fibrous tissue replacement of the infarcted myocardium with only a few of blood vessels (3±1.2) at 7-day post infarction (**FIG. 2****: 4**). RT-PCR and Western blots analysis demonstrated that the Ga-induced revascularization within 24 hours in infarcted myocardium was concomitantly accompanied with the up-regulated gene expressions of VEGF and bFGF in the corresponding heart tissues. The expressions of VEGF and FGF in the Ga- treated myocardium were increased to 1.8 and 2.2 folds respectively (**FIG. 2****: 5 & 6, T**) compared with their expressions in non-treated myocardium of control group (**FIG. 2****: 5 & 6, C**).

### III. Ga-enhanced survival potential and reduction of infarct size

Referring to **FIG. 3****,** seven days after LAD ligation, the myocytes at risk along peri-infarct rim of the controls (**FIG. 3****:** **1**) showed distorted and irregular shapes compared with the myocytes at distal part of the heart. By contrast, the myocytes at the peri-infarct rim of the Ga-treated hearts showed a regular shape (**FIG. 3****:** **2**) and the myofibers remain healthy and not as narrow and thin as in the non-treated heart. With the staining of TUNEL, it was found that number of apoptotic myocytes detected in the Ga-treated left ventricle myocardium (**FIG. 3****:** **2**) was approximately 3-fold lower compared with the non-treated controls (per high power field: 1.70 ±0.18 versus 5.04 ± 0.75, P<0.001; **FIG. 3****:** **1**). These differences were particularly evident within the peri-infarct rim, where the irregularly shaped myocytes in the control hearts had the highest number of apoptotic nuclei, which were stained brown. Most of the apoptotic nuclei were observed at the peri-infarct rim rather than the myocytes distal to the infarct zone. Furthermore, significantly higher density of capillaries surrounded by the myocytes with much less apoptotic nuclei was found in the infarct zone of the Ga-treated hearts. By contrast, significantly lower density of capillaries and more apoptotic nuclei were observed in the non-treated hearts of the control group. Together, these results indicate that the angiogenesis induced by Ga-treatment prevented an extending pro-apoptotic process in both myocytes and endothelial cells, enhanced survival of the viable myocytes and endothelial cells within the peri-infarct zone and consequently improved myocardial function. In order to determine whether the Ga- induced anti-apoptotic effect on the viable myocytes at risk was through expressions of anti-apoptotic proteins, western blots analysis were performed. It was demonstrated that the Ga-induced prevention of extending pro-apoptotic process of heart tissue at risk were concomitantly accompanied by increased gene expressions of key survival factors. The expressions of Akt1 (**FIG. 3: 3****, T**) and Bcl2 (**FIG. 3****: 4, T**) were increased by 3.3 and 2.8 folds respectively compared with the heart tissues in the control group (**Fig. 3** **& 4, C**).

In order to investigate whether the increased survival potential of the viable myocytes and endothelial cells within the peri-infarct zone induced by Ga would result in reduction of infarct size, the infarct sizes of different animal groups were measured. As shown in **FIG. 3****,** the mean proportion of collagenous deposition or scar tissue/ left ventricular myocardium (as defined by Masson's Trichrome stain) was 27.44% in rats treated by Ga (**FIG. 3****: 5**), compared with 39.53% for those in the control group (**FIG. 3****: 6**)14-day post infarction, indicating that Ga-enhanced survival potential of both myocytes and endothelial cells significantly increased the mass of viable myocardium within the anterior free wall of left ventricles. The Ga-treatment-induced reconstitution of damaged coronary vasculature and reduction of the infarct size were accompanied by significant functional improvement, as demonstrated in the echocardiography measurements where, in comparison with non-treated control MI hearts on day 7 and 14 post infarction, ejection fraction (EF) of the Ga-treated MI hearts was significantly higher (55.68± 2.63 vs 49.67 ± 2.78, P = 0.03) on day 7, and significantly increased (60.11 ± 2.66 vs 48.26 ± 2.55, P = 0.001) on day 14. Similarly, fraction shortening (FS) of the Ga-trated MI heart were significantly higher (27.33 ± 1.63 vs 22.17 ± 1.67, P = 0.01) on day 7 and was significantly increased (29.87 ± 2.66 vs 21.35 ± 2.08, P = 0.002) on day 14.

In summary, the above examples demonstrate that Ga is capable of up-regulating the expressions of VEGF and bFGF for early reconstitution of blood supply network, inducing expression of anti-apoptotic proteins-Akt1 and Bcl2 for preventing apoptotic death of cardiomyocytes at risk, and bringing about significant functional improvement of the heart suffering an ischemic event. Thus, Ga provides a new dimension, as a therapeutic angiogenesis medicine, in the treatment of ischemic heart diseases.

### IV Manufacturing Pharmaceutical Compositions and Their Uses in Treating Ischemic Heart Diseases In Mammals

Once the effective chemical compound is identified and partially or substantially pure preparations of the compound are obtained either by isolating the compound from natural resources such as plants or by chemical synthesis, various pharmaceutical compositions or formulations can be fabricated from partially or substantially pure compound using existing processes or future developed processes in the industry. Specific processes of making pharmaceutical formulations and dosage forms (including, but not limited to, tablet, capsule, injection, syrup) from chemical compounds are not part of the invention and people of ordinary skill in the art of the pharmaceutical industry are capable of applying one or more processes established in the industry to the practice of the present invention. Alternatively, people of ordinary skill in the art may modify the existing conventional processes to better suit the compounds of the present invention. For example, the patent or patent application databases provided at USPTO official website contain rich resources concerning making pharmaceutical formulations and products from effective chemical compounds. Another useful source of information is Handbook of Pharmaceutical Manufacturing Formulations, edited by Sarfaraz K. Niazi and sold by Culinary & Hospitality Industry Publications Services.

As used in the instant specification and claims, the term "plant extract" means a mixture of natural occurring compounds obtained via an extracting process from parts of a plant, where at least 10% of the total dried mass is unidentified compounds. In other words, a plant extract does not mean an identified compound substantially purified from the plant. The extracting process typically involves a step of immersing raw plant part(s) in a solvent (commonly, water and/or an organic solvent) for a predetermined length of time, optionally separating the solution from the plant debris and then removing the solvent from the solution, to afford an extract, which may further optionally undergo concentration and/or partial purification. The term "pharmaceutical excipient" means an ingredient contained in a drug formulation that is not a medicinally active constituent. The term "an effective amount" refers to the amount that is sufficient to elicit a therapeutic effect on the treated subject. Effective doses will vary, as recognized by those skilled in the art, depending on the types of diseases treated, route of administration, excipient usage, and the possibility of co-usage with other therapeutic treatment. A person skilled in the art may determine an effective amount in a particular situation using conventional method known in the art.

### V. References

1. Banai S, Jaklitsch M, Casscells W, Shou M, Shrivastav S, Correa R, Epstein S, Unger E. Effects of acidic fibroblast growth factor on normal and ischemic myocardium.Circ Res. 1991;69:76-85.
2. Pu L, Sniderman A, Brassard R, Lachapelle K, Graham A, Lisbona R, Symes J. Enhanced revascularization of the ischemic limb by angiogenic therapy. Circulation. 1993;88:208-215.
3. Folkman J. Clinical Applications of Research on Angiogenesis. N Engl J Med. 1995;333:1757-1763.
4. Risau W. Mechanisms of angiogenesis. 1997;386:671-674.
5. Arras M, Ito WD, Scholz D, Winkler B, Schaper J, Schaper W. Monocyte Activation in Angiogenesis and Collateral Growth in the Rabbit Hindlimb. J. Clin. Invest. 1998;101:40-50.
6. Arras M, Mollnau H, Strasser R, Wenz R, Ito W, Schaper J, Schaper W. The delivery of angiogenic factors to the heart by microsphere therapy. 1998;16:159-162.
7. Schlaudraff K, Schumacher B, von Specht B, Seitelberger R, Schlosser V, Fasol R. Growth of "new" coronary vascular structures by angiogenetic growth factors. Eur J Cardiothorac Surg. 1993;7:637-643.
8. Unger EF, Shou M, Sheffield CD, Hodge E, Jaye M, Epstein SE. Extracardiac to coronary anastomoses support regional left ventricular function in dogs. Am JPhysiol Heart Circ Physiol. 1993;264:H1567-1574.
9. Kocher AA, Schuster MD, Szabolcs MJ, Takuma S, Burkhoff D, Wang J, Homma S, Edwards NM, Itescu S. Neovascularization of ischemic myocardium by human bone-marrow?derived angioblasts prevents cardiomyocyte apoptosis, reduces remodeling and improves cardiac function. Nature Medicine. 2001;7:430 - 436.
10. Liu YH, Yang XP, Nass O, Sabbah HN, Peterson E, Carretero OA. Chronic heart failure induced by coronary artery ligation in Lewis inbred rats. Am J Physiol Heart Circ Physiol. 1997;272:H722-727.
11. Yang F, Liu Y, Yang X, Xu J, Kapke A, Carretero O. Myocardial infarction and cardiac remodelling in mice. Exp Physiol. 2002;87:547-555.
12. Davani S, Marandin A, Mersin N, Royer B, Kantelip B, Herve P, Etievent J-P, Kantelip J-P. Mesenchymal Progenitor Cells Differentiate into an Endothelial Phenotype, Enhance Vascular Density, and Improve Heart Function in a Rat Cellular Cardiomyoplasty Model. Circulation. 2003; 108:253II-258.

## Claims

1. Use of an isolated compound of formula (I) in the manufacture of a medicament for treating coronary heart disease or heart infarction in a mammalian subject. -

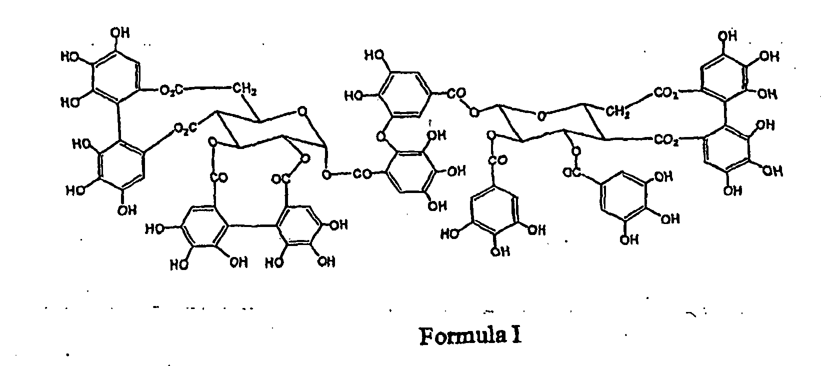

2. Use according to claim 1, wherein said compound exerts a therapeutic effect by revascularization in an infarcted heart tissue of said mammalian subject.

3. Use according to claim 2, where said revascularization occurs within 24 to 72 hours following a treatment with said compound.

4. Use according to claim 1, wherein said coronary heart disease or heart infarction is caused by atherosclerosis of coronary arteries.

5. Use of an isolated compound of formula (I) as defined in claim 1 in the manufacture of a medicament for revascularization in infarcted myocardia of a mammalian subject.

6. Use according to claim 5, wherein said compound up-regulates expression of VEGF and bFGF.

7. Use according to claim 5, wherein said compound is injected directly into tissues in said infarcted myocardia.

8. Use according to claim 5, wherein said compound is delivered to tissues in said infarcted myocardia via oral administration.

9. Use according to claim 5, wherein said compound is delivered to tissues in said infarcted myocardia via subcutaneous injection, intramuscular injection, or intravenous infusion.

10. An isolated compound of formula (I) as defined in claim 1, for use in treating coronary heart disease or heart infarction in a mammalian subject and/or for revascularization in infarcted myocardia of a mammalian subject.

## Patentansprüche

1. Verwendung einer isolierten Verbindung nach Formel (I) bei der Herstellung eines Medikaments zur Behandlung einer koronaren Herzerkrankung oder eines Herzinfarkts bei einem Säugetiersubjekt

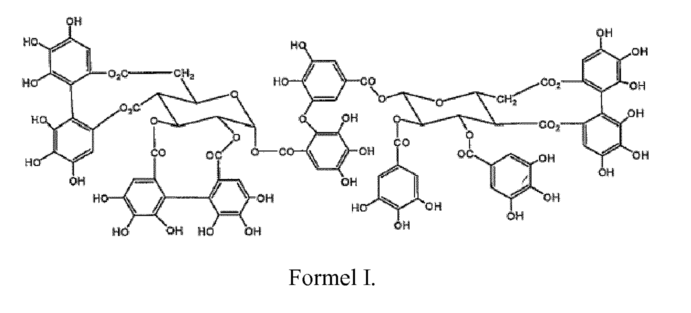

2. Verwendung gemäß Anspruch 1, wobei die Verbindung einen therapeutischen Effekt zeigt durch Revaskularisierung in einem infarzierten Herzgewebe des Säugetiersubj ekts.

3. Verwendung gemäß Anspruch 2, wobei die Revaskularisierung innerhalb von 24 bis 72 Stunden im Anschluss an eine Behandlung mit der Verbindung auftritt.

4. Verwendung gemäß Anspruch 1, wobei die koronare Herzerkrankung oder der Herzinfarkt durch Arteriosklerose von Herzarterien verursacht wird.

5. Verwendung einer isolierten Verbindung nach Formel (I), wie in Anspruch 1 definiert, bei der Herstellung eines Medikaments zur Revaskularisierung in infarzierten Herzmuskeln eines Säugetiersubjekts.

6. Verwendung gemäß Anspruch 5, wobei die Verbindung die Exprimierung von VEGF und bFGF hochreguliert.

7. Verwendung gemäß Anspruch 5, wobei die Verbindung direkt in Gewebe in den infarzierten Herzmuskeln injiziert wird.

8. Verwendung gemäß Anspruch 5, wobei die Verbindung durch orale Verabreichung an Gewebe in den infarzierten Herzmuskeln abgegeben wird.

9. Verwendung gemäß Anspruch 5, wobei die Verbindung an Gewebe in den infarzierten Herzmuskeln über subkutane Injektion, intramuskuläre Injektion oder intravenöse Infusion abgegeben wird.

10. Isolierte Verbindung nach Formel (I), wie in Anspruch 1 definiert, für die Verwendung bei der Behandlung einer koronaren Herzerkrankung oder einem Herzinfarkt bei einem Säugetiersubjekt und/oder für die Revaskularisierung in infarzierten Herzmuskeln eines Säugetiersubj ekts.

## Revendications

1. Utilisation d'un composé isolé de formule (I) dans la fabrication d'un médicament destiné à traiter une coronaropathie ou un infarctus du myocarde chez un sujet mammifère.

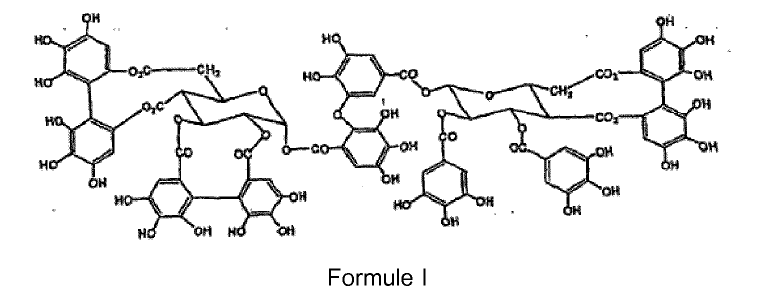

2. Utilisation selon la revendication 1, dans laquelle ledit composé exerce un effet thérapeutique par revascularisation dans un tissu cardiaque infarci dudit sujet mammifère.

3. Utilisation selon la revendication 2, où ladite revascularisation se produit dans les 24 à 72 heures qui suivent un traitement avec ledit composé.

4. Utilisation selon la revendication 1, dans laquelle ladite coronaropathie ou ledit infarctus du myocarde est provoqué(e) par une athérosclérose des artères coronaires.

5. Utilisation d'un composé isolé de formule (I) tel que défini dans la revendication 1 dans la fabrication d'un médicament destiné à une revascularisation dans le myocarde infarci d'un sujet mammifère.

6. Utilisation selon la revendication 5, dans laquelle ledit composé régule positivement l'expression du VEGF et du bFGF.

7. Utilisation selon la revendication 5, dans laquelle ledit composé est injecté directement dans les tissus dans ledit myocarde infarci.

8. Utilisation selon la revendication 5, dans laquelle ledit composé est délivré aux tissus dans ledit myocarde infarci par administration orale.

9. Utilisation selon la revendication 5, dans laquelle ledit composé est délivré aux tissus dans ledit myocarde infarci par injection sous-cutanée, injection intramusculaire, ou perfusion intraveineuse.

10. Composé isolé de formule (I) tel que défini dans la revendication 1, pour une utilisation dans le traitement d'une coronaropathie ou d'un infarctus du myocarde chez un sujet mammifère et/ou pour une revascularisation dans le myocarde infarci d'un sujet mammifère.
